# EUROPEAN PATENT APPLICATION

(11) **EP 4 556 009 A1**
(43) Date of publication of application: **21.05.2025**
(21) Application number: 23839006.6
(22) Date of filing: 13.07.2023
(51) Int. Cl.: A61K 31/522, A61P 35/00

(54) **USE OF IMIDAZOLINONE DERIVATIVE IN COMBINATION WITH RADIOTHERAPY IN TREATMENT OF TUMORS**

(30) Priority: 13.07.2022 CN 202210816032
(71) Applicant: Kangbaida (Sichuan) Biotechnology Co., Ltd., Chengdu City, Sichuan Province (CN)
(72) Inventor: YE, Fei, Chengdu, Sichuan 610000 (CN); WEI, Yonggang, Chengdu, Sichuan 610000 (CN); XU, Xuezhen, Chengdu, Sichuan 610000 (CN); LI, Zhiyong, Chengdu, Sichuan 610000 (CN); SU, Guizhuan, Chengdu, Sichuan 610000 (CN); SUN, Yi, Chengdu, Sichuan 610000 (CN)
(74) Representative: Carpmaels & Ransford LLP
(86) International application number: PCT/CN2023/107149
(87) International publication number: WO 2024/012516

(57) **Abstract**

The present invention provides the use of a compound represented by general formula (I) in combination with radiotherapy in a medicament for treating cancers

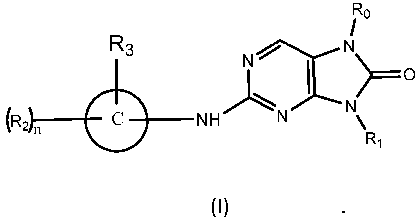

## Description

### TECHNICAL FIELD

The present invention relates to the use of an imidazolinone derivative for the treatment of a tumor in combination with radiotherapy.

### BACKGROUND ART

Radiotherapy is one of the most important methods for the comprehensive treatment of malignant tumors, and the neoadjuvant therapy based on conventional long-term radiotherapy has become a standard regimen for treating various malignant tumors. However, long-term clinical studies have found that the responses of patients with malignant tumors to radiotherapy vary greatly. Although some patients can achieve complete pathological remission at the end of a treatment cycle, there are still some patients who can not benefit from long-term treatment, and a few even develop worsening progress. Studies have found that the enhanced DNA damage repair mechanism due to radiotherapy is an important reason for the tolerance mechanism of tumors to radiotherapy.

DNA double-strand break (DSB) is a highly harmful form of DNA damage in cells, and DNA double-strand breaks that are not repaired in time are closely associated with the canceration of cells. Non-homologous end-joining (NHEJ) is one of the main pathways for the repair of DNA double-strand breaks in cells. In NHEJ, the DSB end is first recognized and bound by Ku70/80 and then binds to a DNA-dependent protein kinase catalytic subunit (DNA-PKcs) to form a DNA-dependent protein kinase (DNAPK), i.e., an NHEJ initiation complex (DNAPK). Subsequently, two DNAPKs bind to the ends of damaged DNA while recruiting subsequent NHEJ repair factors (XRCC4 and XLF) and DNA ligase IV (LigIV) to repair the damaged DNA. Studies have shown that DNA-PK activity is associated with drug resistance due to radiotherapy, such that the killing effect of radiotherapy on tumor cells can be improved by inhibiting the DNA-PK activity in tumor cells. However, there are still no effective DNA-PK inhibitor drugs on the market. Therefore, developing a potent DNA-PK inhibitor as a radiosensitizing drug for tumor treatment has important clinical significance.

WO 2021209055 discloses the use of an imidazolinone derivative in the preparation of a drug for use in the treatment of a cancer, in which the compounds described in the specification have high selectivity and significant inhibitory activity against DNA-PK.

Herein, the inventors of the present invention aim to increase the sensitivity of tumors to radiotherapy by combining the prior art with radiotherapy to overcome the deficiencies of the prior art.

### SUMMARY OF THE INVENTION

An objective of the present invention is to provide the use of an imidazolinone derivative for the treatment of a tumor in combination with radiotherapy, so as to overcome the deficiencies of the prior art.

In one or more embodiments of the present application, there is provided the use of a compound of general formula (I), or a stereoisomer, solvate, prodrug, metabolite, pharmaceutically acceptable salt or co-crystal thereof as an active ingredient for the treatment of a tumor in combination with radiotherapy: 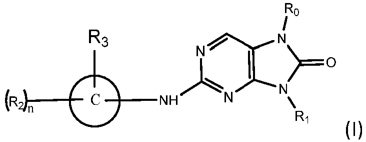 wherein 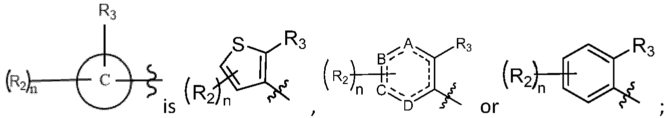
-̅ -̅ -̅ is a single bond or double bond;
in 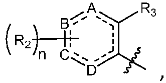 A, B, C, D are each independently C or N, and at least one of A, B, C and D is N;
R₀ is H, C₁₋₆ alkyl or cyclopropyl, wherein the C₁₋₆ alkyl is optionally further substituted with one or more substituents selected from halogen and deuterium;
R₁ is 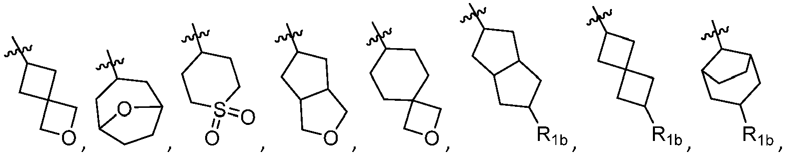 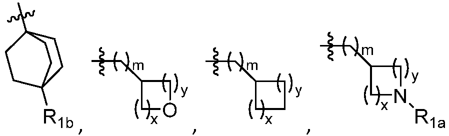 or pyridyl, and R₁ is optionally further substituted with 1 or 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R₂ is H, cyano, =O, carboxyl, -C(=O)NR₂ₐR_{2b}, C₁₋₆ alkoxy, C₁₋₆ alkyl, halogen, -S(=O)₂R₂ₐ or -C(=O)OC₁₋₆ alkyl, wherein the C₁₋₆ alkyl, -C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1, 2 or 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3;
with the provisos that
when 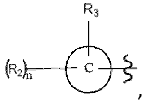 R₀, R₂, and R₃ simultaneously satisfy the following conditions, R₁ is not 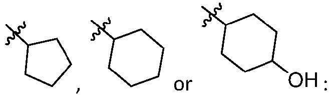 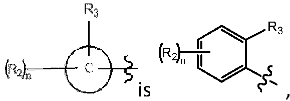 n is 1, R₀ is H or methyl, R₂ is methoxy or -S(=O)₂Me, and R₃ is methyl.

In one or more embodiments of the present application, R₁ is 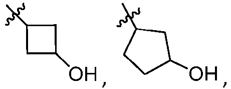 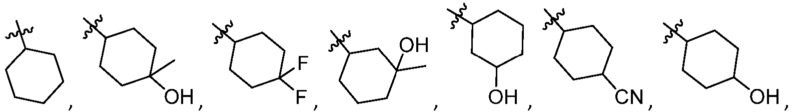 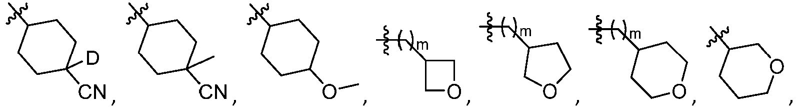 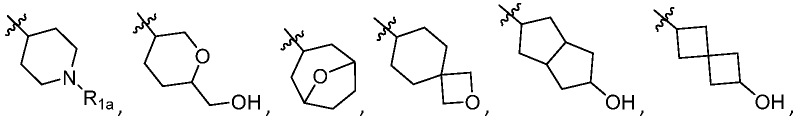 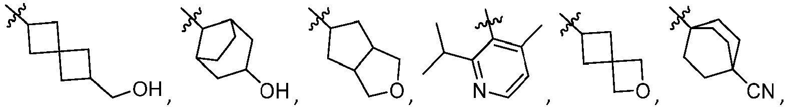 or 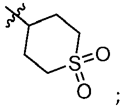
R₁ₐ is H or C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the - C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D or halogen;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1; and
n is 0, 1 or 2;
with the provisos that
when 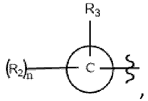 R₀, R₂, and R₃ simultaneously satisfy the following conditions, R₁ is not 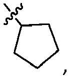 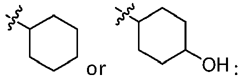 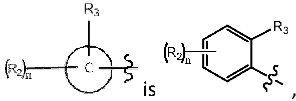 n is 1, R₀ is H or methyl, R₂ is methoxy or -S(=O)₂Me, and R₃ is methyl.

In one or more embodiments of the present application, 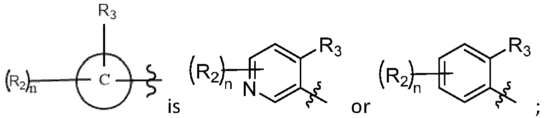
R₀ is H, C₁₋₄ alkyl or cyclopropyl, wherein the C₁₋₄ alkyl is optionally further substituted with one or more substituents selected from halogen and D;
R₁ is 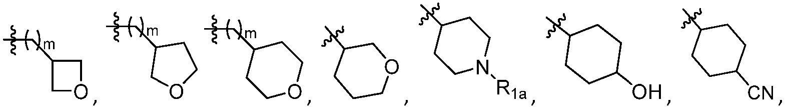 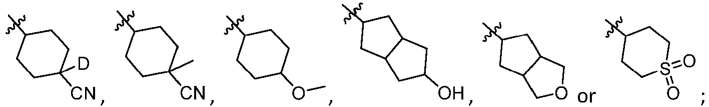
R₁ₐ is H, C₁₋₆ alkyl or -C(=O)C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the - C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D and halogen;
alternatively, R₂ₐ and R_{2b} together with the atoms to which they are attached form a 5- to 6-membered heterocyclyl, which contains 1 to 3 heteroatoms selected from N, O and S and is also optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1; and
n is 0, 1 or 2;
with the provisos that
when 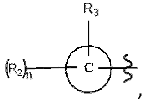 R₀, R₂, and R₃ simultaneously satisfy the following conditions, R₁ is not 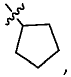 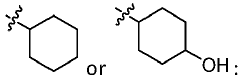
when 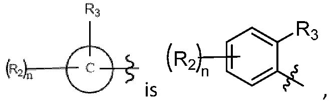 n is 1, R₀ is H or methyl, R₂ is methoxy or -S(=O)₂Me, and R₃ is methyl.

In one or more embodiments of the present application, there is provided the use of a compound of general formula (II), or a stereoisomer, solvate, prodrug, metabolite, pharmaceutically acceptable salt or co-crystal thereof as an active ingredient for the treatment of a tumor in combination with radiotherapy: 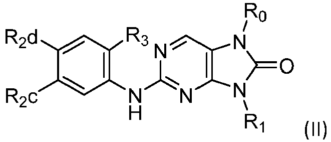 wherein
R₀ is H, C₁₋₆ alkyl or cyclopropyl, wherein the C₁₋₆ alkyl is optionally further substituted with one or more substituents selected from halogen and deuterium;
R₁ is 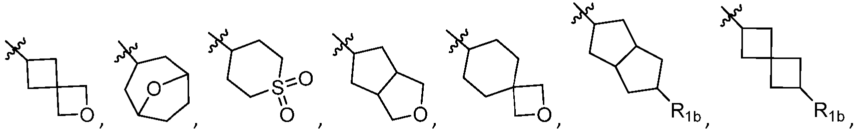 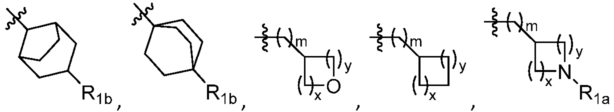 or pyridyl, and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R_{2c} is H, cyano, halogen or C₁₋₆ alkoxy;
R_{2d} is H, cyano, carboxyl, -C(=O)NR₂ₐR_{2b}, C₁₋₆ alkyl, halogen, -S(=O)₂R₂ₐ or -C(=O)OC₁₋₆ alkyl, wherein the C₁₋₆ alkyl and -C(=O)OC₁₋₆ alkyl is optionally substituted with one or more substituents selected from halogen and deuterium;
R₂ₐ and R_{2b} are H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3;
with the provisos that
when 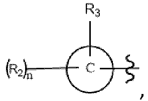 R₀, R₂, and R₃ simultaneously satisfy the following conditions, R₁ is not 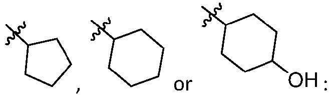 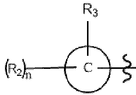 is selected from 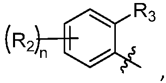 n is 1, R₀ is H or methyl, R₂ is methoxy or - S(=O)₂Me, and R₃ is methyl.

In one or more embodiments of the present application, there is provided the use of a compound of general formula (III), or a stereoisomer, solvate, prodrug, metabolite, pharmaceutically acceptable salt or co-crystal thereof as an active ingredient for the treatment of a tumor in combination with radiotherapy: 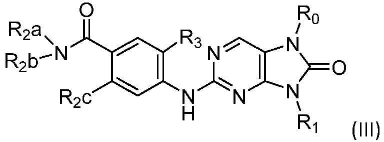 wherein
R₀ is H, C₁₋₆ alkyl or cyclopropyl, wherein the C₁₋₆ alkyl is optionally further substituted with one or more substituents selected from halogen and deuterium;
R₁ is 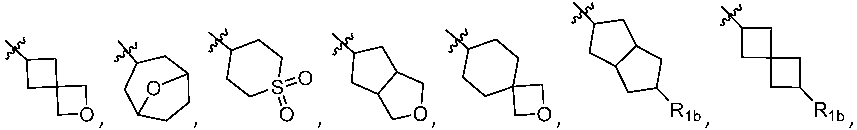 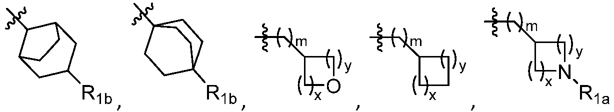 or pyridyl, and R₁ is optionally further substituted with 1 to 2 substituents selected from D, halogen, cyano, hydroxyl, C₁₋₆ alkyl and C₁₋₆ alkoxy;
R_{1b} is H, OH, cyano, or hydroxyl substituted C₁₋₆ alkyl;
R₂ₐ and R_{2b} are each independently H, C₁₋₆ alkyl, or 3- to 5-membered cycloalkyl, wherein the C₁₋₆ alkyl is optionally further substituted with one or more substituents selected from D or halogen;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R_{2c} is H, cyano, halogen or C₁₋₆ alkoxy, wherein the C₁₋₆ alkoxy is optionally substituted with one or more deuterium;
R₃ is halogen or C₁₋₆ alkyl, wherein the C₁₋₆ alkyl is optionally further substituted with 1 to 3 substituents selected from D or halogen;
m is 0 or 1;
n is 0, 1 or 2; and
x and y are each independently 1, 2 or 3.

In one or more embodiments of the present application, there is provided the use of a compound of general formula (IV), or a stereoisomer, solvate, prodrug, metabolite, pharmaceutically acceptable salt or co-crystal thereof as an active ingredient for the treatment of a tumor in combination with radiotherapy: 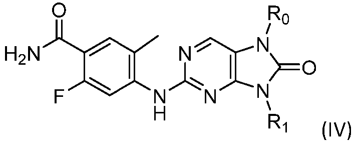 wherein
R₀ is H, C₁₋₆ alkyl or cyclopropyl, wherein the C₁₋₆ alkyl is optionally further substituted with one or more substituents selected from halogen and deuterium; and
R₁ is -(CH)ₘ-4- to 7-membered carbocyclyl, -(CH)ₘ-4- to 7-membered heterocyclyl, -(CH)ₘ-8- to 12-membered bridged ring, -(CH)ₘ-7- to 12-membered spiro ring, wherein the -(CH)ₘ-4- to 7-membered carbocyclyl, -(CH)ₘ-4- to 7-membered heterocyclyl, -(CH)ₘ-8- to 12-membered bridged ring, or -(CH)ₘ-7- to 12-membered spiro ring is optionally further substituted with one or more substituents selected from hydroxy, cyano, halogen, =O, C₁₋₆ alkyl, C₁₋₆ alkoxy, and hydroxy substituted C₁₋₆ alkyl.

In one or more embodiments of the present application,
R₀ is H, C₁₋₆ alkyl or cyclopropyl, wherein the C₁₋₆ alkyl is optionally further substituted with one or more substituents selected from halogen and deuterium;
R₁ is 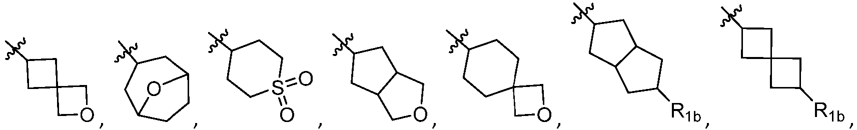 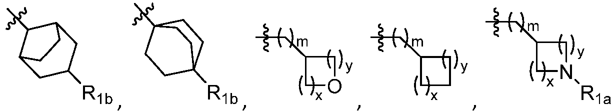 or pyridyl, and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
m is 0 or 1; and
x and y are each independently 1, 2 or 3.

In one or more embodiments of the present application,
R₀ is C₁₋₄ alkyl, wherein the C₁₋₄ alkyl is optionally further substituted with one or more substituents selected from halogen and deuterium; and
R₁ is

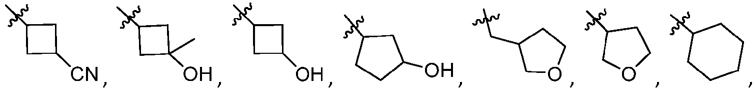

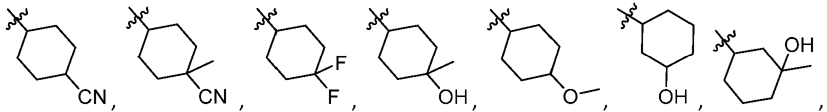

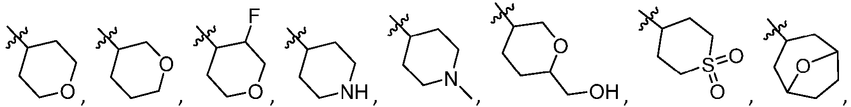

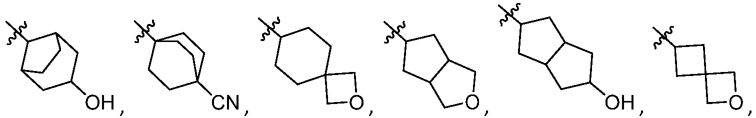

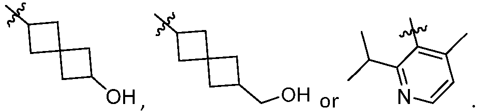

In one or more embodiments of the present application, the active ingredient is selected from:

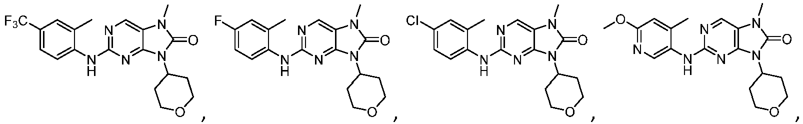

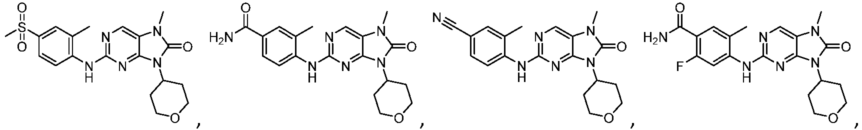

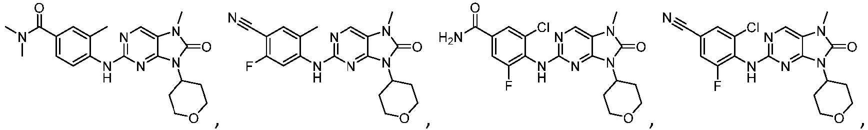

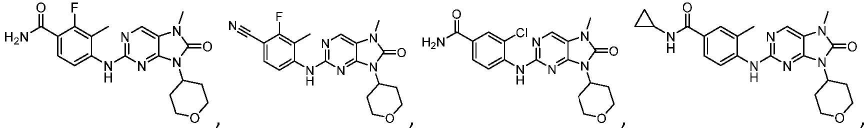

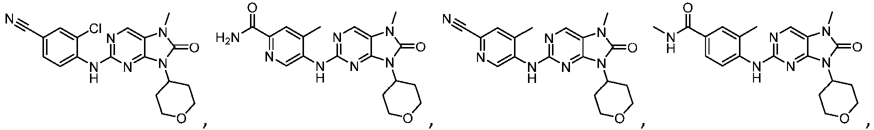

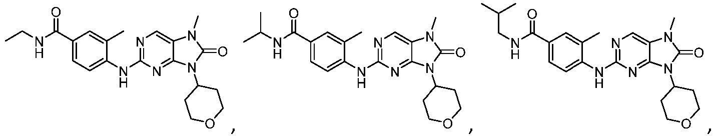

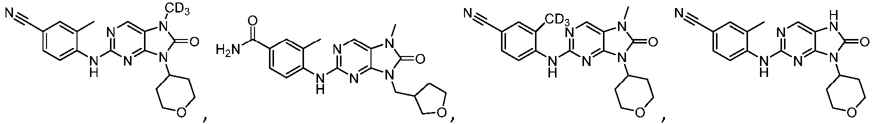

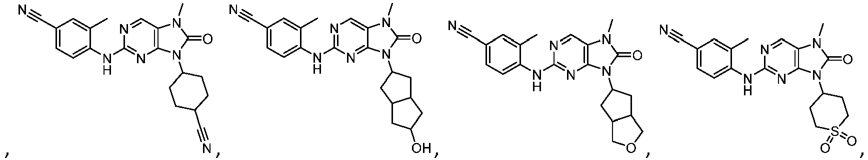

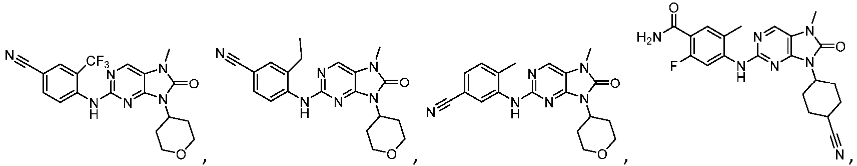

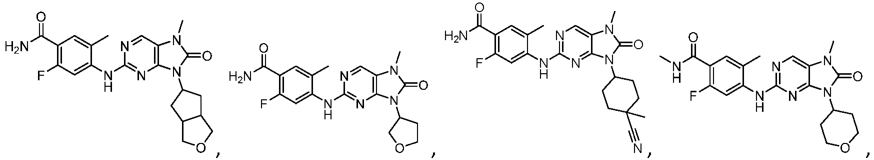

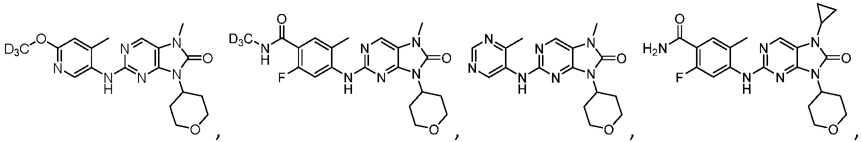

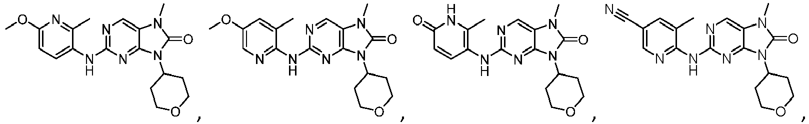

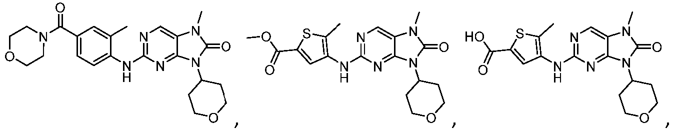

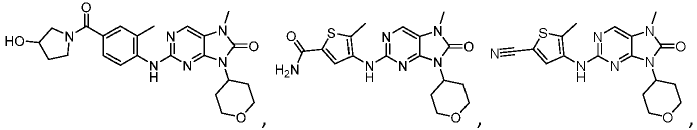

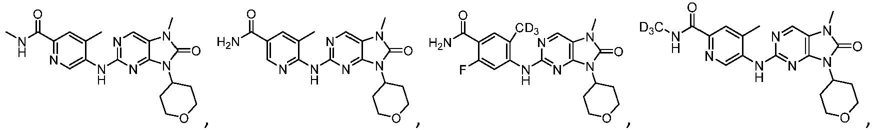

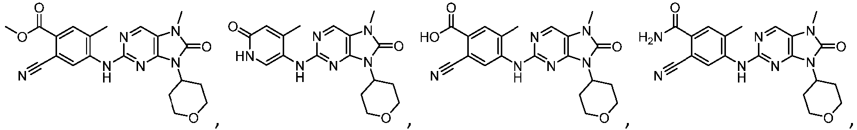

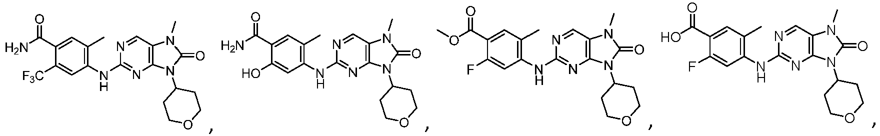

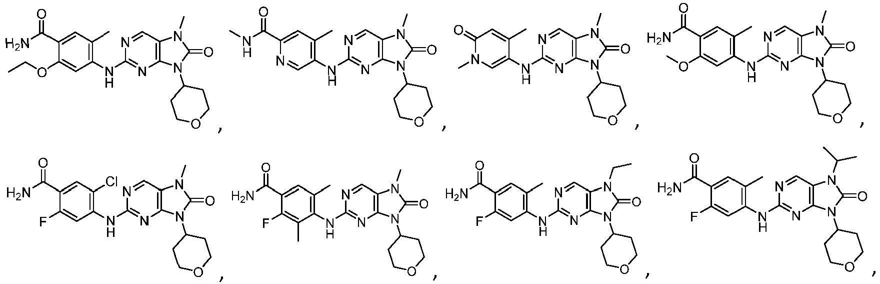

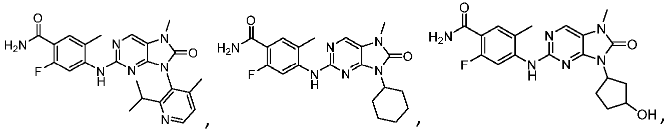

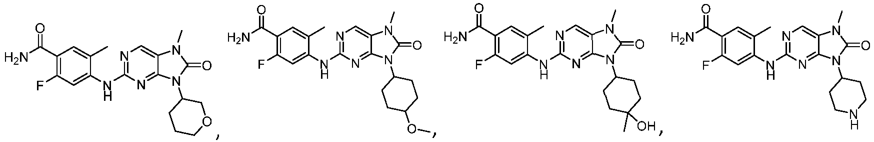

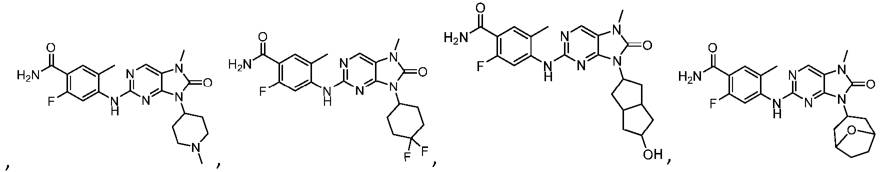

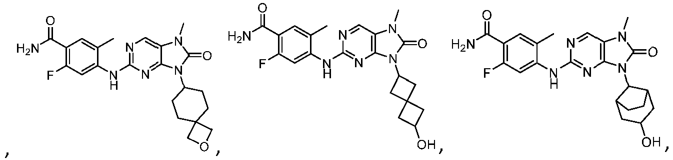

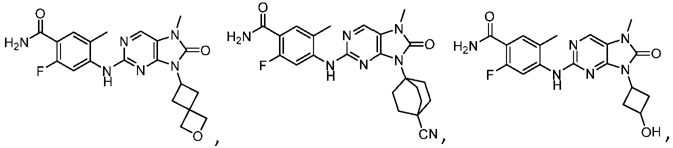

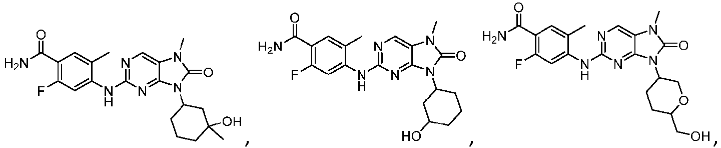

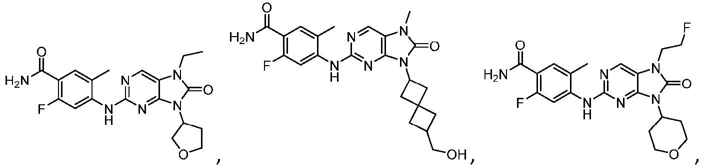

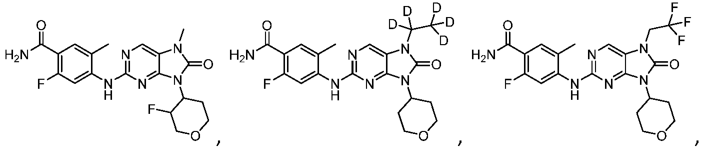

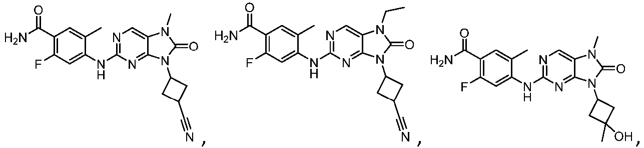

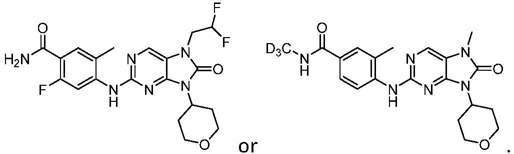

In one or more embodiments of the present application, the active ingredient, when used in combination with radiotherapy, has the effect of increasing the sensitivity of tumors to radiotherapy.

In one or more embodiments of the present application, the tumor is selected from a solid tumor.

### BRIEF DESCRIPTION OF THE DRAWINGS

FIG. 1 shows a compound A;
FIG. 2 shows a graph of the trend of the volume of tumor in a BALB/c mouse model bearing a CT26 transplanted tumor; and
FIG. 3 shows a graph of the trend of body weight change in a BALB/c mouse model bearing a CT26 transplanted tumor.

### DETAILED DESCRIPTION OF EMBODIMENTS

The implementation process and beneficial effects of the present invention are described in detail below through specific examples, which are intended to help readers better understand the essence and characteristics of the present invention, and are not intended to limit the scope of implementation of the present invention.

The present invention will be described in further detail below with reference to the accompanying drawings.

The compound A in the examples is compound 62 of WO 2021209055 and is prepared according to the preparation method therefor.

### Inhibition experiment using BALB/c mouse model bearing CT26 transplanted tumor

### 1. Experimental steps:

Female BALB/c mice were selected to constitute a CT26 subcutaneous transplanted tumor model by subcutaneous injection of tumor cells. When the mean tumor volume reached 150-200 mm³, mice were divided into 3 experimental groups: Vehicle, IR, and (Compound A (20 mpk) + IR), respectively, with 8 animals per group and 24 animals in total for grouping experiments. Each irradiated group was given an irradiation dose of 2 Gy for 5 consecutive days, the administration group was given the corresponding drug once a day for 39 consecutive days, and the Vehicle group was given a vehicle.

### 2. Assay method

2.1 Tumor volume: during a drug treatment cycle, the long diameter (a) and short diameter (b) of tumor tissue were measured using an electronic vernier caliper to calculate the volume of the tumor (tumor volume = 0.5 × a × b²).

2.2 Body weight of mice: mice were weighed at least twice a week during a drug treatment cycle.

### 3. Experimental results

### 3.1 Tumor volume results

**Table 1. Tumor volume statistics (Mean ± SEM, mm³)**

| **Treatment** | **D0** | **D4** | **D7** | **D11** | **D14** | **D18** |
|---|---|---|---|---|---|---|
| Vehicle | 167 ± 25 | 307 ± 59 | 475 ± 80 | 804 ± 137 | 1230 ± 255 | 2073 ± 428 |
| IR | 168 ± 24 | 298 ± 56 | 342 ± 62 | 315 ± 80** | 234 ± 61*** | 267 ± 83*** |
| Compound A (20 mpk) + IR | 168 ± 22 | 260 ± 41 | 284 ± 47* | 253 ± 56*** | 155 ± 28*** | 101 ± 33***# |

| **Treatment** | **D21** | **D25** | **D28** | **D32** | **D35** | **D39** |
|---|---|---|---|---|---|---|
| Vehicle | / | / | / | / | / | / |
| IR | 361 ± 115 | 520 ± 148 | 724 ± 196 | 1068 ± 309 | 1520 ± 418 | 2251 ± 634 |
| Compound A (20 mpk) + IR | 93 ± 34# | 169 ± 71# | 288 ± 105# | 462 ± 148# | 605 ± 189# | 828 ± 247# |

| | | | | | | |
|---|---|---|---|---|---|---|
| Notes: the day of grouping is D0; * indicates P ≤ 0.05 compared to animals in the Vehicle group; ** indicates P ≤ 0.01 compared to animals in the Vehicle group; *** indicates P ≤ 0.001 compared to animals in the Vehicle group; # indicates P ≤ 0.05 compared to animals in the IR group; mpk is mg/kg; and at D18, since the tumor in some mice in the Vehicle group was too large, the mice in this group were euthanized considering animal ethics issues. | | | | | | |

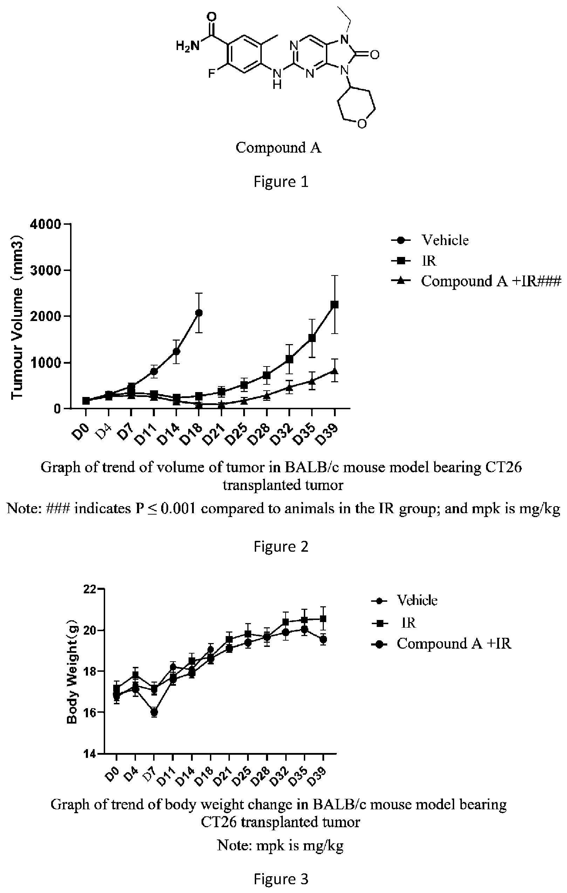

As shown in Table 1 and FIG. 2, the tumor volume of the group treated with compound A in combination with IR was significantly reduced compared to the Vehicle and IR groups, and after four days of treatment, the difference in volume between the group treated with compound A in combination with IR and the IR group gradually increased, indicating that the compound A can significantly enhance the anti-tumor efficacy of radiotherapy.

### 3.2 Body weight results

**Table 2 Body weight statistics**

| **Treatment** | **D0** | **D4** | **D7** | **D11** | **D14** | **D18** |
|---|---|---|---|---|---|---|
| Vehicle | 16.7 ± 0.3 | 17.3 ± 0.3 | 17.1 ± 0.3 | 18.2 ± 0.3 | 18.1 ± 0.3 | 19.1 ± 0.3 |
| IR | 17.2 ± 0.4 | 17.8 ± 0.4 | 17.2 ± 0.3 | 17.7 ± 0.4 | 18.5 ± 0.4 | 18.7 ± 0.3 |
| Compound A (20 mpk) + IR | 16.8 ± 0.3 | 17.1 ± 0.3 | 16.0 ± 0.2 | 17.6 ± 0.2 | 17.9 ± 0.2 | 18.6 ± 0.2 |

| **Treatment** | **D21** | **D25** | **D28** | **D32** | **D35** | **D39** |
|---|---|---|---|---|---|---|
| Vehicle | / | / | / | / | / | / |
| IR | 19.6 ± 0.4 | 19.8 ± 0.5 | 19.7 ± 0.4 | 20.4 ± 0.5 | 20.5 ± 0.5 | 20.6 ± 0.6 |
| Compound A (20 mpk) + IR | 19.1 ± 0.2 | 19.4 ± 0.3 | 19.7 ± 0.2 | 19.9 ± 0.4 | 20.1 ± 0.3 | 19.6 ± 0.3 |

| | | | | | | |
|---|---|---|---|---|---|---|
| Notes: mpk is mg/kg; and at D18, since the tumor in some mice in the Vehicle group was too large, the mice in this group were euthanized considering animal ethics issues. | | | | | | |

As shown in Table 2 and FIG. 3, the body weight of mice in the Vehicle group was stable during the experiment; the body weight of the IR group was slightly decreased during irradiation and began to rise after irradiation; and at the end of the experiment at D39, there is no significant difference in mean body weight between the IR group and the group treated with compound A in combination with IR, indicating that the treatment of compound A in combination with radiotherapy had no effect on body weight gain of animals and the animals had good tolerance.

Specific embodiments are described in detail in the description of the present invention. A person skilled in the art should recognize that the embodiments described above are exemplary and cannot be construed as limiting the present invention. Additionally, a person skilled in the art can make several improvements and modifications to the present invention without departing from the principle of the present invention, and the technical solutions obtained based on these improvements and modifications also fall within the scope of protection of the claims of the present invention.

## Claims

1. Use of a compound represented by formula (I), or a stereoisomer, solvate, prodrug, metabolite, pharmaceutically acceptable salt or co-crystal thereof as an active ingredient for the treatment of a tumor in combination with radiotherapy: wherein 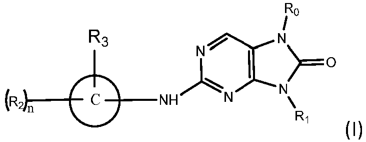 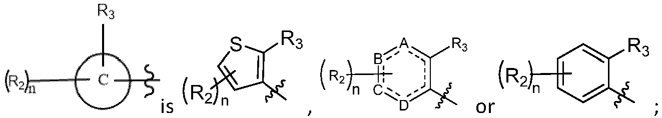
-̅ -̅ -̅ is a single bond or double bond;
in 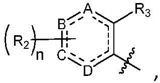
A, B, C, D are each independently C or N, and at least one of A, B, C and D is N;
R₀ is H, C₁₋₆ alkyl or cyclopropyl, wherein the C₁₋₆ alkyl is optionally further substituted with one or more substituents selected from halogen and deuterium;
R₁ is 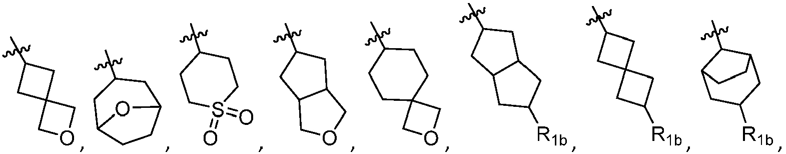 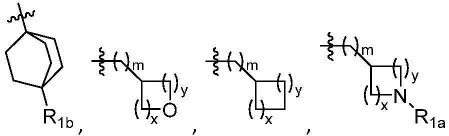
or pyridyl, and R₁ is optionally further substituted with 1 or 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R₂ is H, cyano, =O, carboxyl, -C(=O)NR₂ₐR_{2b}, C₁₋₆ alkoxy, C₁₋₆ alkyl, halogen, -S(=O)₂R₂ₐ or - C(=O)OC₁₋₆ alkyl, wherein the C₁₋₆ alkyl, -C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1, 2 or 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3;
with the provisos that
when 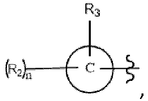
R₀, R₂, and R₃ simultaneously satisfy the following conditions, R₁ is not 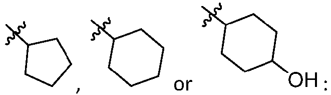 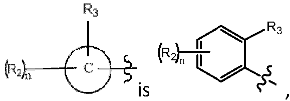
n is 1, R₀ is H or methyl, R₂ is methoxy or -S(=O)₂Me, and R₃ is methyl.

2. The use according to claim 1, wherein the active ingredient is the compound of formula (I), or a stereoisomer, solvate, prodrug, metabolite, pharmaceutically acceptable salt or co-crystal thereof, wherein
R₁ is 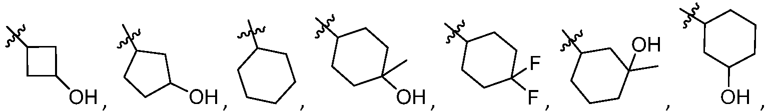 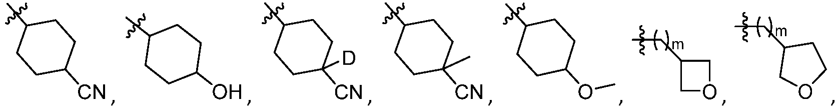 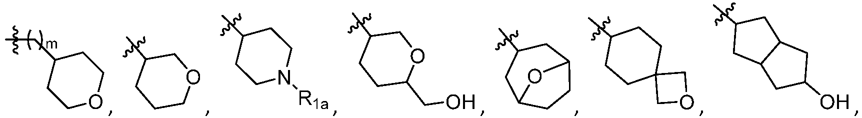 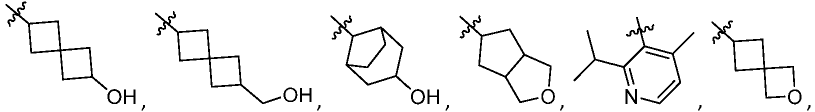 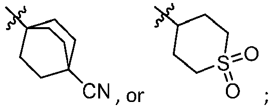
R₁ₐ is H or C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the - C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D or halogen;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1; and
n is 0, 1 or 2;
with the provisos that
when 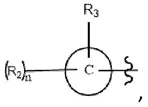
R₀, R₂, and R₃ simultaneously satisfy the following conditions, R₁ is not 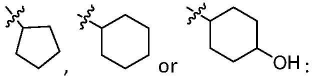 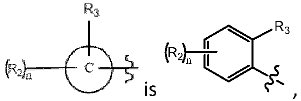
n is 1, R₀ is H or methyl, R₂ is methoxy or -S(=O)₂Me, and R₃ is methyl.

3. The use according to claim 1, wherein the active ingredient is the compound of formula (I), or a stereoisomer, solvate, prodrug, metabolite, pharmaceutically acceptable salt or co-crystal thereof, wherein 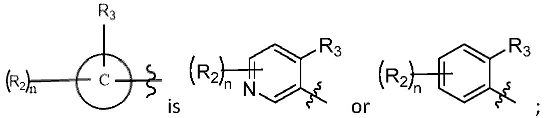
R₀ is H, C₁₋₄ alkyl or cyclopropyl, wherein the C₁₋₄ alkyl is optionally further substituted with one or more substituents selected from halogen and D;
R₁ is 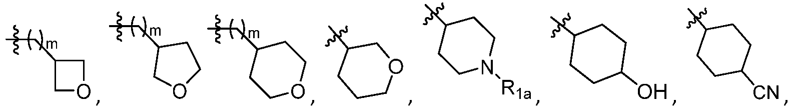 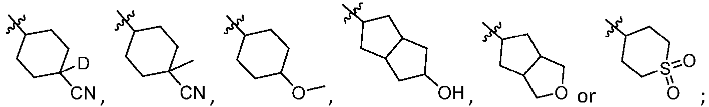
R₁ₐ is H, C₁₋₆ alkyl or -C(=O)C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the - C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D and halogen;
alternatively, R₂ₐ and R_{2b} together with the atoms to which they are attached form a 5- to 6-membered heterocyclyl, which contains 1 to 3 heteroatoms selected from N, O and S and is also optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1; and
n is 0, 1 or 2;
with the provisos that
when 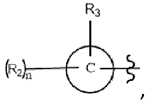
R₀, R₂, and R₃ simultaneously satisfy the following conditions, R₁ is not 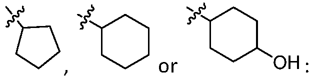
when 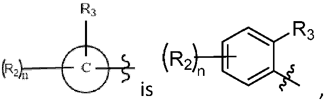
n is 1, R₀ is H or methyl, R₂ is methoxy or -S(=O)₂Me, and R₃ is methyl.

4. The use according to claim 1, wherein the active ingredient is selected from a compound of formula (II), or a stereoisomer, solvate, prodrug, metabolite, pharmaceutically acceptable salt or co-crystal thereof: 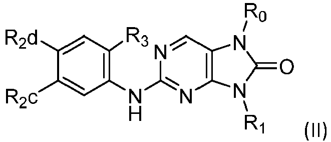 wherein
R₀ is H, C₁₋₆ alkyl or cyclopropyl, wherein the C₁₋₆ alkyl is optionally further substituted with one or more substituents selected from halogen and deuterium;
R₁ is 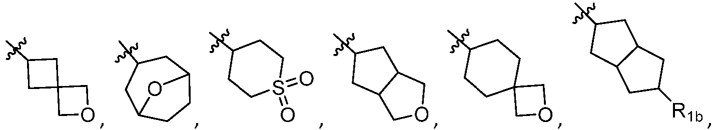 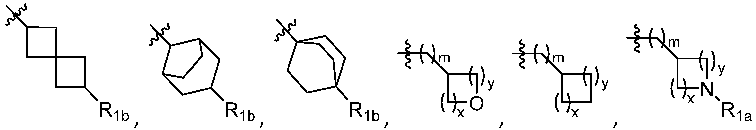
or pyridyl, and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R_{2c} is H, cyano, halogen or C₁₋₆ alkoxy;
R_{2d} is H, cyano, carboxyl, -C(=O)NR₂ₐR_{2b}, C₁₋₆ alkyl, halogen, -S(=O)₂R₂ₐ or -C(=O)OC₁₋₆ alkyl, wherein the C₁₋₆ alkyl and -C(=O)OC₁₋₆ alkyl is optionally substituted with one or more substituents selected from halogen and deuterium;
R₂ₐ and R_{2b} are H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3;
with the provisos that
when 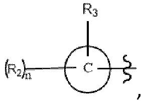
R₀, R₂, and R₃ simultaneously satisfy the following conditions, R₁ is not 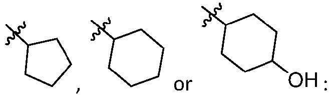 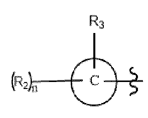
is selected from 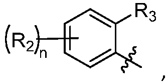
n is 1, R₀ is H or methyl, R₂ is methoxy or - S(=O)₂Me, and R₃ is methyl.

5. The use according to claim 4, wherein the active ingredient is selected from a compound of formula (III), or a stereoisomer, solvate, prodrug, metabolite, pharmaceutically acceptable salt or co-crystal thereof: 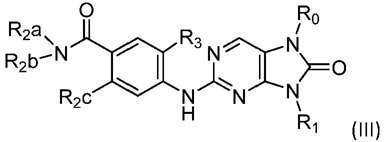 wherein
R₀ is H, C₁₋₆ alkyl or cyclopropyl, wherein the C₁₋₆ alkyl is optionally further substituted with one or more substituents selected from halogen and deuterium;
R₁ is 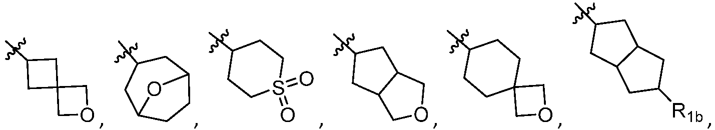 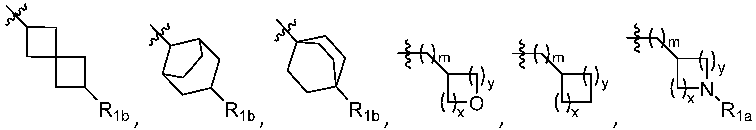
or pyridyl, and R₁ is optionally further substituted with 1 to 2 substituents selected from D, halogen, cyano, hydroxyl, C₁₋₆ alkyl and C₁₋₆ alkoxy;
R_{1b} is H, OH, cyano, or hydroxyl substituted C₁₋₆ alkyl;
R₂ₐ and R_{2b} are each independently H, C₁₋₆ alkyl, or 3- to 5-membered cycloalkyl, wherein the C₁₋₆ alkyl is optionally further substituted with one or more substituents selected from D or halogen;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R_{2c} is H, cyano, halogen or C₁₋₆ alkoxy, wherein the C₁₋₆ alkoxy is optionally substituted with one or more deuterium;
R₃ is halogen or C₁₋₆ alkyl, wherein the C₁₋₆ alkyl is optionally further substituted with 1 to 3 substituents selected from D or halogen;
m is 0 or 1;
n is 0, 1 or 2; and
x and y are each independently 1, 2 or 3.

6. The use according to claim 1, wherein the active ingredient is selected from a compound of formula (IV), or a stereoisomer, solvate, prodrug, metabolite, pharmaceutically acceptable salt or co-crystal thereof: 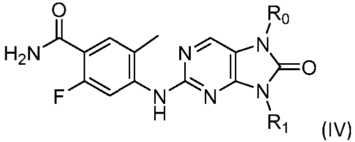 wherein
R₀ is H, C₁₋₆ alkyl or cyclopropyl, wherein the C₁₋₆ alkyl is optionally further substituted with one or more substituents selected from halogen and deuterium; and
R₁ is -(CH)ₘ-4- to 7-membered carbocyclyl, -(CH)ₘ-4- to 7-membered heterocyclyl, -(CH)ₘ-8- to 12-membered bridged ring, -(CH)ₘ-7- to 12-membered spiro ring, wherein the -(CH)ₘ-4- to 7-membered carbocyclyl, -(CH)ₘ-4- to 7-membered heterocyclyl, -(CH)ₘ-8- to 12-membered bridged ring, or -(CH)ₘ-7- to 12-membered spiro ring is optionally further substituted with one or more substituents selected from hydroxy, cyano, halogen, =O, C₁₋₆ alkyl, C₁₋₆ alkoxy, and hydroxy substituted C₁₋₆ alkyl.

7. The use according to claim 6, wherein the active ingredient is selected from the compound of formula (IV), or a stereoisomer, solvate, prodrug, metabolite, pharmaceutically acceptable salt or co-crystal thereof, wherein
R₀ is H, C₁₋₆ alkyl or cyclopropyl, wherein the C₁₋₆ alkyl is optionally further substituted with one or more substituents selected from halogen and deuterium;
R₁ is 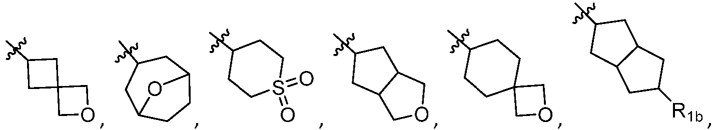 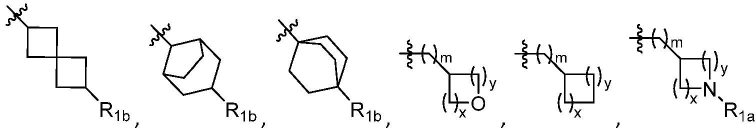
or pyridyl, and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
m is 0 or 1; and
x and y are each independently 1, 2 or 3.

8. The use according to claim 7, wherein the active ingredient is the compound of formula (IV), or a stereoisomer, solvate, prodrug, metabolite, pharmaceutically acceptable salt or co-crystal thereof, wherein
R₀ is C₁₋₄ alkyl, wherein the C₁₋₄ alkyl is optionally further substituted with one or more substituents selected from halogen and deuterium; and
R₁ is

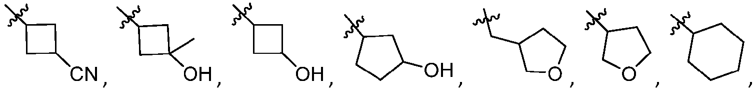

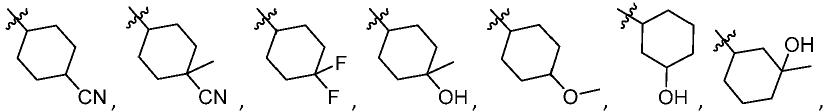

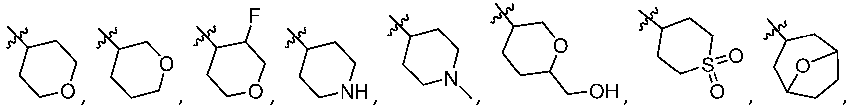

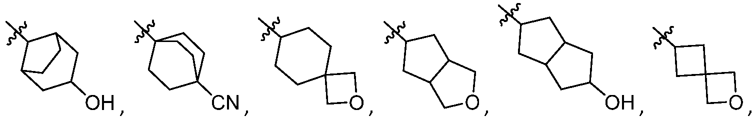

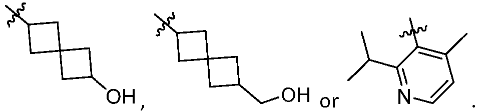

9. The use according to any one of claims 1-8, wherein the active ingredient is selected from:

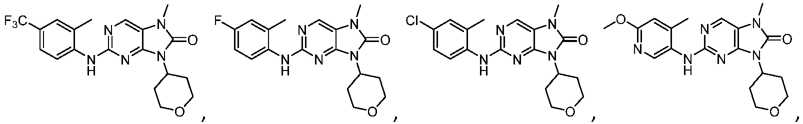

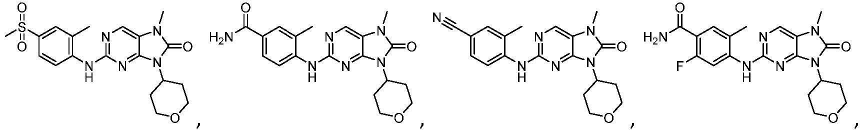

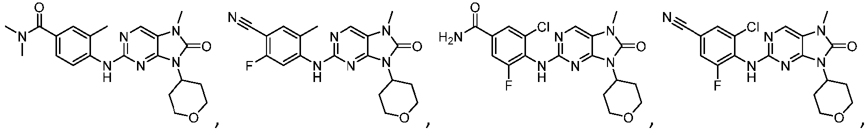

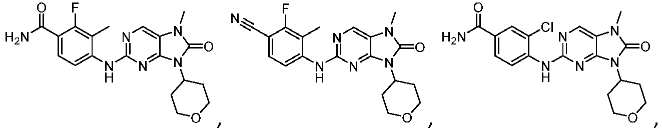

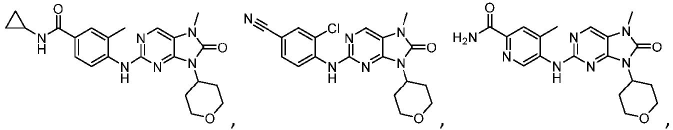

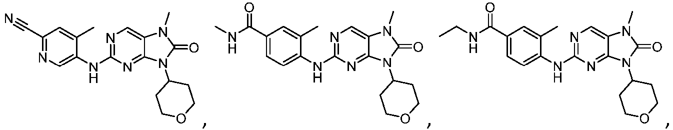

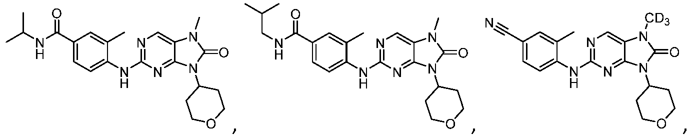

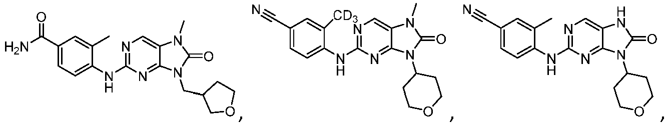

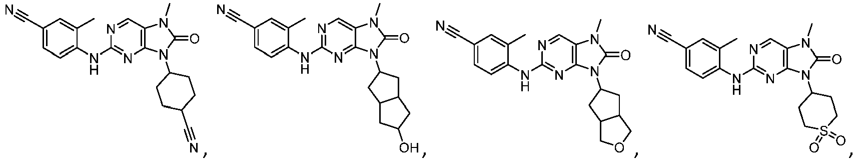

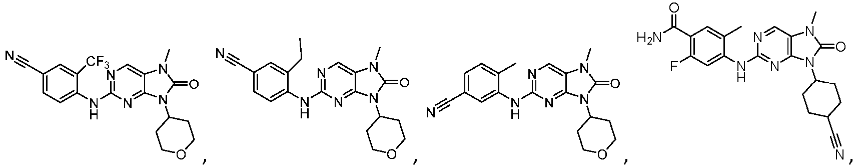

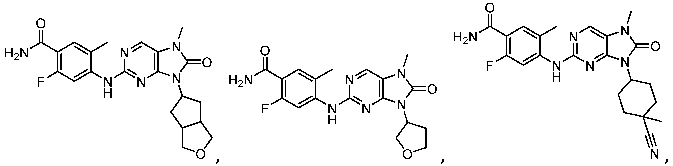

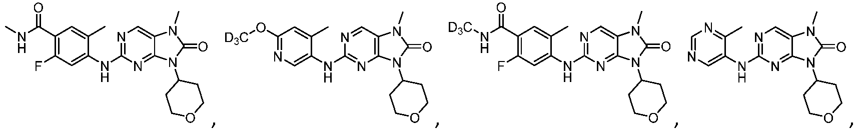

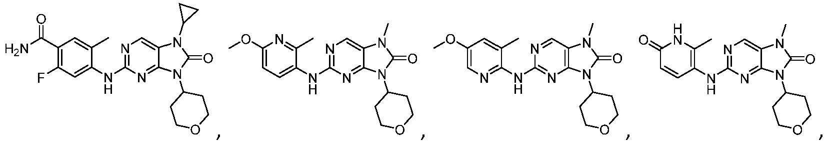

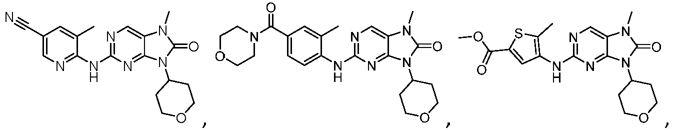

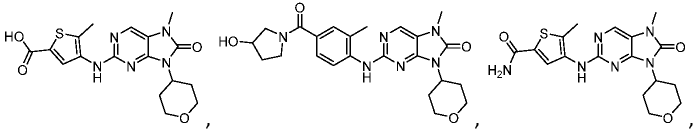

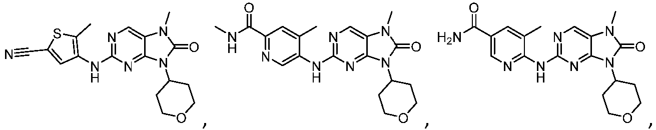

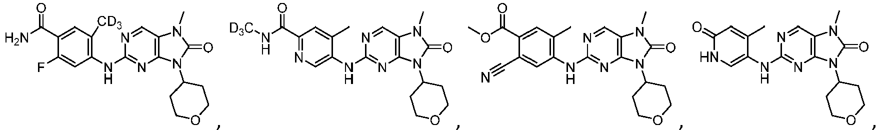

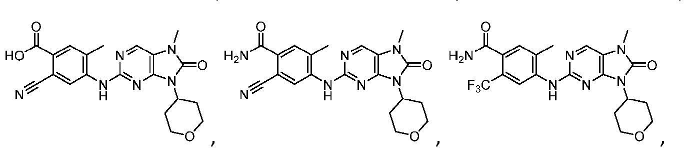

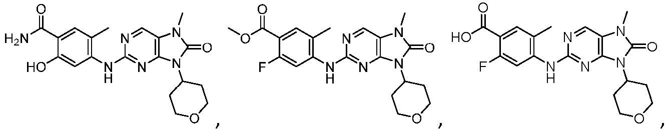

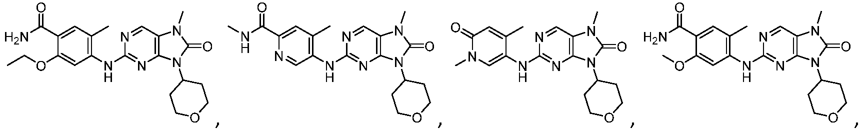

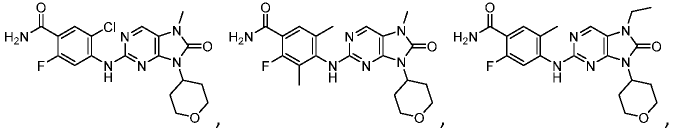

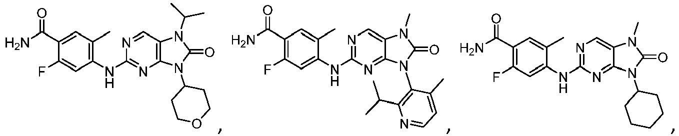

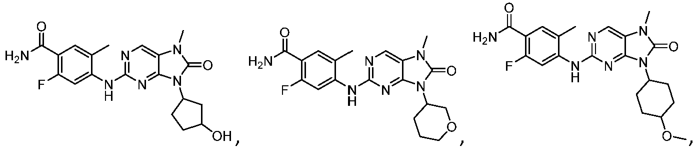

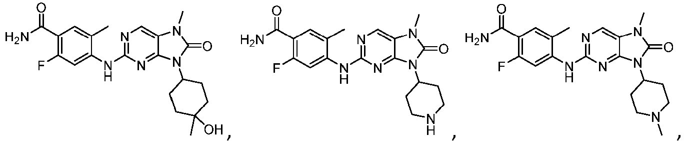

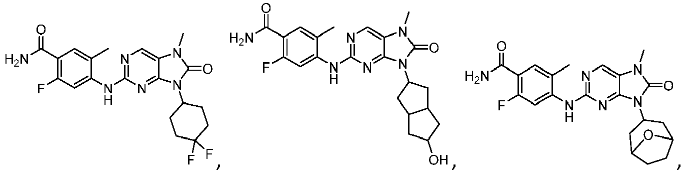

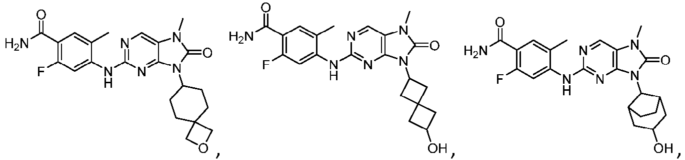

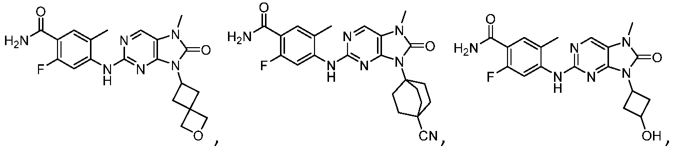

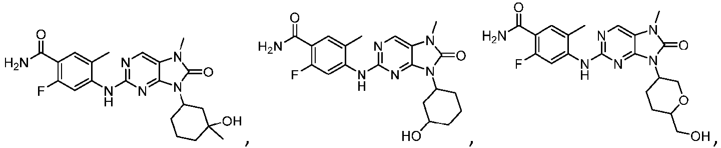

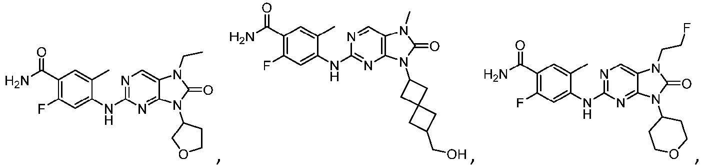

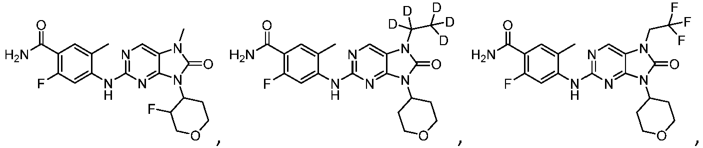

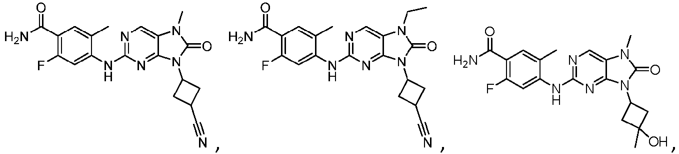

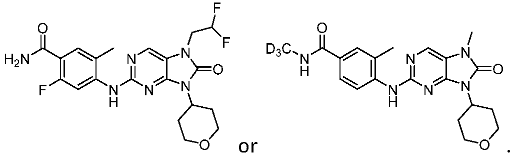

10. The use according to any one of claims 1 to 9, wherein the tumor is selected from a solid tumor.
